# EUROPÄISCHE PATENTSCHRIFT

(11) **EP 1 236 037 B1**
(45) Veröffentlichungstag und Bekanntmachung des Hinweises auf die Patenterteilung: **10.10.2007**
(21) Anmeldenummer: 00954828.0
(22) Anmeldetag: 06.09.2000
(51) Int. Cl.: G01N 27/00, G01N 33/00

(54) **SENSOR IN EINEM GEHÄUSE**
SENSOR ACCOMMODATED IN A HOUSING
CAPTEUR PLACE DANS UN BOITIER

(30) Priorität: 30.11.1999 CH 219199
(43) Veröffentlichungstag der Anmeldung: 04.09.2002
(73) Patentinhaber: Sensirion AG, 8712 Stäfa (CH)
(72) Erfinder: MAYER, Felix, CH-8057 Zürich (CH); HORNUNG, Mark, CH-8049 Zürich (CH)
(74) Vertreter: Blum, Rudolf Emil
(86) Internationale Anmeldenummer: PCT/IB2000/001250
(87) Internationale Veröffentlichungsnummer: WO 2001/040784

(56) Entgegenhaltungen:
- WO-A-98/27411
- US-A- 5 388 443

## Beschreibung

### Hintergrund

Die Erfindung betrifft einen Sensor zum Ausmessen von Parametern eines Fluids, insbesondere zum Ermitteln von Stoffen in einem Gas, und ein Verfahren zur Herstellung eines solchen Sensors gemäss Oberbegriff der unabhängigen Ansprüche.

### Stand der Technik

Typische Beispiele für Sensoren dieser Art sind Feuchtesensoren und CO₂-Sensoren. Die Gehäusung solcher Sensoren ist recht aufwendig, da der Halbleiterchip einerseits in Kontakt mit dem zu messenden Medium stehen muss, andrerseits möglichst gut von der Umgebung geschützt werden sollte.

In WO 98/27411 wird ein Sensor mit Halbleiterchip beschrieben, der in Flip-Chip-Technik auf einem Substrat angeordnet ist. Im Substrat ist eine Öffnung vorgesehen, über welche eine auf dem Chip integrierte Messanordnung zugänglich ist. Zwischen Substrat und Chip wird eine Dichtungsmasse eingefüllt. Auch bei dieser Anordnung ist der Chip jedoch von hinten frei zugänglich.

### Darstellung der Erfindung

Es ist Aufgabe der vorliegenden Erfindung, einen Sensor bzw. ein Verfahren der eingangs genannten Art bereitzustellen, der bzw. die dieses Problem zumindest teilweise lindert.

Diese Aufgabe wird von den unabhängigen Ansprüchen gelöst.

Das Gehäuse des erfindungsgemässen Sensors ist also mit einem Barrierenteil ausgerüstet, der sich gegen den Halbleiterchip erstreckt und diesen in mindestens zwei Teile unterteilt. Der erste Teil, in welchem sich die eigentliche Messanordnung des Halbleiterchips befindet, ist über mindestens eine Öffnung im Gehäuse nach aussen verbunden. In diesem Teil, der mit dem zu messenden Medium in Kontakt steht, kann die Messung durchgeführt werden. Der zweite Teil wird mindestens teilweise, vorzugsweise ganz, von einer ausgehärteten Abdichtmasse bedeckt. Diese schützt den Halbleiterchip in diesem Teil vor Umwelteinflüssen. Der Barrierenteil bildet eine Barriere für die Abdichtmasse zwischen den beiden Teilen. Bei der Herstellung kann die noch flüssige Abdichtmasse in den zweiten Teil des Gehäuses eingebracht werden, wobei der Barrierenteil verhindert, dass die Masse in den ersten Teil eintritt.

Der Barrierenteil ist als Steg ausgeführt, der vorzugsweise einen geraden, gebogenen oder abgewinkelten Verlauf besitzt.

Vorzugsweise nähert sich der Barrierenteil bis auf einen dünnen Spalt dem Halbleiterchip, wobei der Spalt mit Abdichtmasse gefüllt ist, um eine gute Abdichtung zu erreichen. Die Spaltbreite ist möglichst so gewählt, dass die noch flüssige Masse bei der Herstellung durch Kapillarkraft in den Spalt eingezogen wird.

Der Halbleiterchip wird auf einem Substrat angeordnet, welches vorzugsweise ebenfalls mindestens teilweise mit der Abdichtmasse in Kontakt steht. Auf diese Weise kann das Substrat mit dem Gehäuse verbunden werden. Die Abdichtmasse kann auch verwendet werden, um die elektrischen Verbindungen bzw. Bonddrähte zwischen dem Halbleiterchip und dem Substrat und/oder eine integrierte elektrischen Schaltung auf dem Halbleiterchip zu schützen.

Das Gehäuse kann verschiedenste Form aufweisen. Vorzugsweise besteht es aus einem leitenden Kunststoff, oder mit leitfähigem Material beschichtetem Kunststoff, um den Halbleiterchip vor elektrischen Störungen zu schützen.

Im Bereich des zweiten Teils sollte das Gehäuse eine Öffnung zum Einfüllen der Abdichtmasse aufweisen.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 ein Längsschnitt durch eine Ausführung eines erfindungsgemässen Sensors,
Fig. 2 eine Draufsicht auf den Sensor nach Fig. 1,
Fig. 3 einen Schnitt entlang Linie III-III von Fig. 1,
Fig. 4 einen Schnitt entlang Linie IV-IV von Fig. 1,
Fig. 5 einen Schnitt entlang Linie V-V von Fig. 1 und
Fig. 6 eine teilweise angeschnittene Ansicht mehrerer Sensoren vor dem Zersägen.

### Weg zur Ausführung der Erfindung

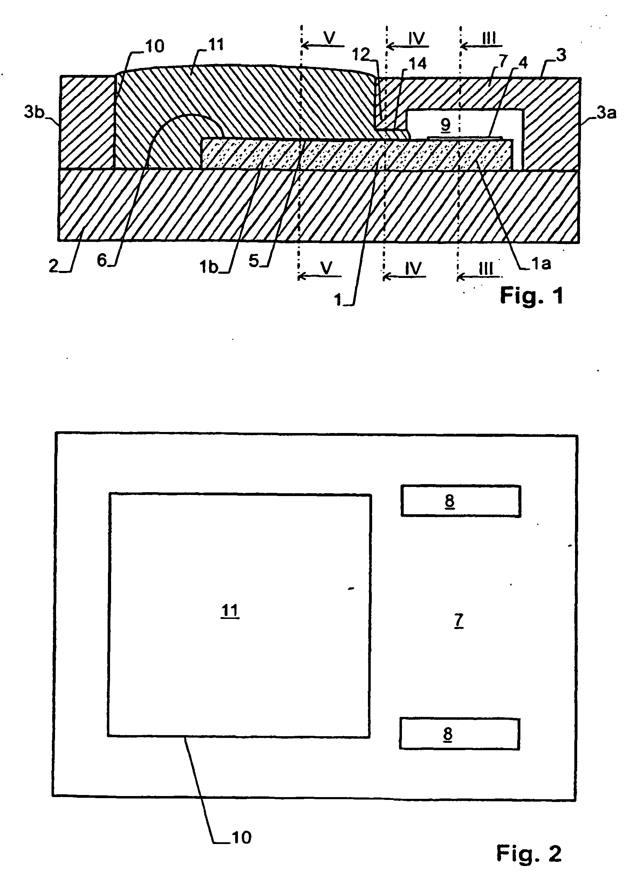

In Fig. 1 bis 5 wird eine bevorzugte Ausführung eines Feuchtesensors gezeigt. Er besitzt einen Halbleiterchip 1, der auf einem Substrat 2 liegt. Auf dem Substrat 2 ist ein Gehäuse 3 aus Kunststoff angeordnet. Um den Halbleiterchip 1 vor elektrischen Störungen zu schützen, kann der Kunststoff schwach leitend sein, oder er kann mit leitfähigem Material beschichtet sein.

Auf dem Halbleiterchip befindet sich eine Messanordnung 4, z.B. bestehend aus einer Polymerschicht oder einer anderen feuchtigkeitsempfindlichen Schicht 1) zwischen zwei Elektroden, deren elektrische Eigenschaften sich abhängig von der Feuchtigkeit der Umgebung ändern oder 2) auf schwingenden Balkenstrukturen, deren Schwingungsfrequenz sich mit der Eigenschaft der Umgebung ändert. Ferner ist auf dem Halbleiterchip 1 eine integrierte Schaltung 5 angeordnet, in der eine Vorverarbeitung oder vollständige Verarbeitung der Messresultate stattfindet.

Der Halbleiterchip 1 ist über Bonddrähte 6 mit dem Substrat 2 verbunden. Das Substrat ist als Printplatte ausgebildet. Es trägt gegebenenfalls Bausteine zur weiteren Verarbeitung der Signale vom Halbleiterchip 1. Ausserdem besitzt es Leitungen, die an (nicht gezeigte) Anschlusakontakte an dessen Unterseite führen.

Das Substrat 2 und das Gehäuse 3 haben, wie insbesondere aus Fig. 2 ersichtlich, eine deckungsgleiche rechteckige Aussenform. Das Gehäuse 3 besitzt vier Seitenwände 3a, 3b, 3c, 3d, welche auf dem Substrat 2 aufliegen und sich seitlich um die Aussenkanten des Halbleiterchips 1 herum erstrecken. Der in Fig. 1 rechte Teil des Gehäuses 3 ist mit einer Deckwand 7 ausgestattet, in welcher sich zwei Schlitzöffnungen 8 befinden. Die Schlitzöffnungen 8 verbinden einen Hohlraum 9 zur Aufnahme der Messanordnung 4 unter der Deckwand 7 mit der Umgebung.

Der in Fig. 1 linke Teil des Gehäuses 3 besitzt eine grosse Öffnung 10 zum Einfüllen einer aushärtbaren Abdichtmasse 11, welche einen Teil des Halbleiterchips 1 und die Bonddrähte 6 bedeckt bzw. einschliesst und diese schützt. Die Abdichtmasse kontaktiert ausserdem das Gehäuse 3 und das Substrat 2 und bildet eine adhäsive Verbindung zwischen diesen beiden Teilen.

Der linke und der rechte Teil des Gehäuses sind von einem Steg 12 unterteilt, der an die Deckwand 7 anschliesst und gegenüber den Seitenwänden 3c, 3d etwas zurückversetzt ist, so dass zwischen dem Steg 12 und dem Substrat ausreichend Platz für den Halbleiterchip 1 bleibt. Der Steg 12 erstreckt sich von oben gegen den Halbleiterchip 1 und teilt diesen in einen ersten (rechten) und einen zweiten (linken) Teil 1a bzw. 1b. Im linken Teil 1a liegt die Messanordnung 4, im rechten Teil 1b befinden sich die integrierte Schaltung 5 und die Anschlussstellen für die Bonddrähte 6. Der erste Teil 1a grenzt an den Hohlraum 9, der zweite Teil 1b wird von der Abdichtmasse 11 bedeckt.

Zwischen dem Steg 12 und dem Halbleiterchip 1 verläuft ein dünner Spalt 14. Er ist so dimensioniert, dass er eine starke Kapillarkraft auf die im Herstellungsprozess noch flüssige Abdichtmasse ausübt, so dass diese in den Spalt 14 einfliesst. Der Spalt 14 ist jedoch so dünn bemessen, dass die starke Änderung der Kapillarkraft am Ende der Barrierenteils die Abdichtmasse 11 hindert, in den Hohlraum 9 einzutreten.

Der Steg 12 bildet also einen Barrierenteil des Gehäuses, der die Abdichtmasse vom ersten Teil des Halbleiters fernhält.

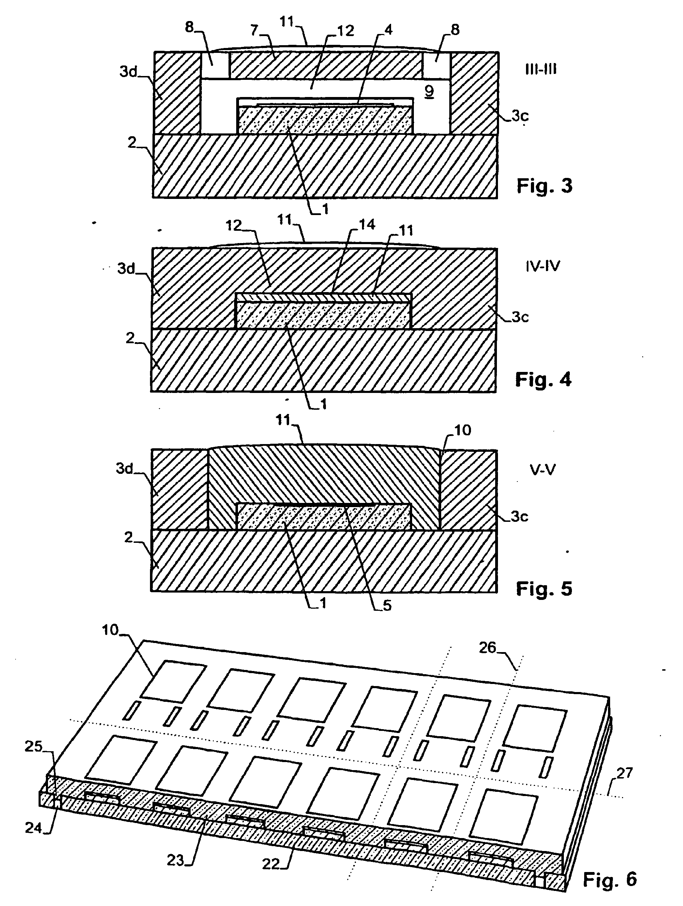

Bei der Herstellung werden, wie in Fig. 6 dargestellt, mehrere Sensoren gleichzeitig gefertigt. Hierzu wird eine Substratmatrix 22 aus mehreren, zusammenhängenden Substraten 2 verwendet, auf welcher in den vorgegebenen Positionen die Halbleiterchips 1 und gegebenenfalls weitere Komponenten befestigt werden.

Sodann wird eine Gehäusematrix 23 auf die Substratmatrix 22 gesetzt. Die Gehäusematrix 23 z.B. ist ein einstückiges Spritzgussteil, in welchem mehrere Gehäuse 2 ausgeformt sind. Seitliche Führungslöcher 24 bzw. Führungsstifte 25 dienen als Positioniermittel zur exakten Positionierung der Gehäusematrix 23 auf der Substratmatrix 22.

Nun wird die Abdichtungsmasse 11 in die Öffnungen 10 eingefüllt. Dabei braucht auf eine genaue Positionierung der Abdichtmasse 11 nicht geachtet zu werden, da die Gehäusematrix 23 und insbesondere die Stege 12 die Abdichtmasse 11 an die richtigen Stellen leiten. Insbesondere die durch die Geometrie des Gehäuses bedingten Änderungen der Oberflächenspannung der Vergussmasse verhinderen ein unerwünschtes Bedecken der Sensorstruktur.

Nach dem Aushärten der Abdichtmasse sind die Sensoren fertig. Bevor sie voneinander getrennt werden, wird die ganze in Fig. 6 dargestellte Sensormatrix kalibriert, indem sie in eine Referenzumgebung bekannter Luftfeuchtigkeit eingebracht wird. Somit kann in einem einzigen Schritt eine Vielzahl von Sensoren geeicht werden.

Schliesslich wird die Anordnung zersägt, so dass die einzelnen Sensoren entstehen. Zwei der entsprechenden Sägelinien 26, 27 sind in Fig. 6 angedeutet.

Wie eingangs erwähnt eignet sich die Erfindung nicht nur für Feuchtesensoren, sondern auch für andere Sensortypen zum Ausmessen von Gas- bzw. Flüssigkeitsparametern, wie z.B. CO₂-Sensoren.

Während in der vorliegenden Anmeldung bevorzugte Ausführungen der Erfindung beschrieben sind, ist klar darauf hinzuweisen, dass die Erfindung nicht auf diese Beschränkt ist und in auch anderer Weise innerhalb des Umfangs der folgenden Ansprüche ausgeführt werden kann.

## Patentansprüche

1. Sensor, insbesondere zum Ermitteln von Stoffen in einem Gas, mit einer Messanordnung (4) auf einem Halbleiterchip (1), wobei der Halbleiterchip (1) mit Bonddrähten (6) verbunden und von einem Gehäuse (3) geschützt ist, wobei der Halbleiterchip (1) in mindestens zwei Teile (1a, 1b) unterteilt ist, wobei der erste Teil (1a) über mindestens eine Öffnung (8) im Gehäuse (3) nach aussen verbunden ist und die Messanordnung (4) trägt, und der zweite Teil (1b) mindestens teilweise von einer ausgehärteten Abdichtmasse (11) bedeckt ist, wobei das Gehäuse (3) und der Halbleiterchip (1) auf einem Substrat (2) angeordnet sind und der Halbleiterchip (1) mit dem Substrat (2) über die Bonddrähte verbunden ist, das Gehäuse (3) eine Deckwand (7), quer zur Deckwand stehende Seitenwände (3a - 3d) und einen Barrierenteil (12) in Form eines Stegs zwischen gegenüberliegenden Seitenwänden (3c, 3d) .. umfasst, der Barrierenteil (12) sich gegen den Halbleiterchip (1) erstreckt, den Halbleiterchip (1) in die mindestens zwei Teile (1a, 1b) teilt und eine Barriere für die Abdichtmasse bildet, und wobei die Messanordnung (4) zwischen dem Substrat (2) und der Deckwand (7) angeordnet und zur Deckwand (7) hin gerichtet ist.

2. Sensor nach Anspruch 1, **dadurch gekennzeichnet, dass** sich der Barrierenteil (12) bis auf einen Spalt (14) dem Halbleiterchip (1) nähert, und dass der Spalt (14) durch die Abdichtmasse (11) gefüllt ist.

3. Sensor nach einem der vorangehenden Ansprüche, **dadurch gekennzeichnet, dass** die Abdichtmasse (11) mindestens einen Teil des Substrats (2) bedeckt.

4. Sensor nach Anspruch 3, **dadurch gekennzeichnet, dass** die Abdichtmasse (11) das Substrat (2) mit dem Gehäuse (3) verbindet.

5. Sensor nach einem der Ansprüche 3 oder 4, **dadurch gekennzeichnet, dass** die Abdichtmasse (11) die Bonddrähte (6) zwischen dem Halbleiterchip (1) und dem Substrat (2) und vorzugsweise auch eine auf dem Halbleiterchip (1) angeordnete integrierte Schaltung (5) bedeckt.

6. Sensor nach einem der vorangehenden Ansprüche, **dadurch gekennzeichnet, dass** der Halbleiterchip (1) auf einem Substrat (2) angeordnet ist und die Seitenwände (3a - 3d) auf einem Randbereich des Substrats (2) stehen und mindestens ein Teil des Barrierenteils (12) im Vergleich zu den Seitenwänden zurückversetzt ist, um Platz für den Halbleiterchip (1) zu bieten.

7. Sensor nach einem der vorangehenden Ansprüche, **dadurch gekennzeichnet, dass** das Gehäuse (3) einteilig ist, und insbesondere dass es aus einem leitenden Kunststoff ist.

8. Sensor nach einem der vorangehenden Ansprüche, **dadurch gekennzeichnet, dass** das Gehäuse (3) aus Kunststoff ist und mit einer leitfähigen Schicht beschichtet ist.

9. Sensor nach einem der vorangehenden Ansprüche, **dadurch gekennzeichnet, dass** das Gehäuse (3) eine Einfüllöffnung (10) für die Abdichtmasse (11) aufweist.

10. Sensor nach einem der vorangehenden Ansprüche, **dadurch gekennzeichnet, dass** der Barrierenteil (12) als Steg im Gehäuse ausgestaltet ist.

11. Sensor nach einem der vorangehenden Ansprüche, **dadurch gekennzeichnet, dass** das Gehäuse sich um die Aussenkanten des Halbleiterchips herum erstreckt.

12. Verfahren zur Herstellung eines Sensors nach einem der vorangehenden Ansprüche, wobei der Sensor eine Messanordnung (4) auf einem Halbleiterchip (1) aufweist, mit Bonddrähten (6) verbunden wird, und von einem Gehäuse (3) geschützt wird, wobei der Halbleiterchip (1) in mindestens zwei Teile (1a, 1b) unterteilt ist, wobei der erste Teil (1a) über mindestens eine Öffnung (8) im Gehäuse (3) nach aussen verbunden wird und die Messanordnung (4) trägt, und der zweite Teil (1b) mindestens teilweise von einer aushärtenden Abdichtmasse (11) bedeckt wird, wobei der Halbleiterchip mit den Bonddrähten (6) mit einem Substrat (2) verbunden wird, das Gehäuse (3) eine Deckwand (7), quer zur Deckwand stehende Seitenwände (3a - 3d) und einen Barrierenteil (12) in Form eines Stegs zwischen gegenüberliegenden Seitenwänden (3c, 3d) umfasst, der Barrierenteil (12) sich gegen den Halbleiterchip (1) erstreckt und zum Verhindern einer Ausbreitung der Abdichtmasse (11) in den ersten Teil (1a) verwendet wird, und wobei die Messanordnung (4) zwischen dem Substrat (2) und der Deckwand (7) angeordnet und zur Deckwand (7) hin gerichtet ist.

13. Verfahren nach Anspruch 12, **dadurch gekennzeichnet, dass** die aushärtende Abdichtmasse (11) durch Kapillarkraft in einen Spalt (14) zwischen dem Barrierenteil (12) und dem Halbleiterchip (1) eingebracht wird.

14. Verfahren nach einem der Ansprüche 12 oder 13, **dadurch gekennzeichnet, dass** mehrere Halbleiterchips (1) auf einer Substratmatrix (22) angeordnet werden, dass eine Gehäusematrix (23) aus mehreren, miteinander verbundenen Gehäusen auf die Halbleiterchips gebracht wird, dass im Bereich jedes Sensors Abdichtmasse (11) eingebracht wird, und dass die Substratmatrix (22) zusammen mit der Gehäusematrix (23) zerteilt wird um die einzelnen Sensoren zu bilden.

15. Verfahren nach Anspruch 14, **dadurch gekennzeichnet, dass** die Sensoren vor dem Zerteilen in eine Referenzumgebung gebracht und kalibriert werden.

## Claims

1. Sensor, in particular for detecting substances in a gas, with a measuring section (4) on a semiconductor chip (1), wherein the semiconductor chip (1) is connected to bond wires (6) and is protected by a housing (3), wherein the semiconductor chip (1) is divided into at least two parts (1a, 1b), wherein the first part (1a) is connected with the outside through at least one opening (8) in the housing (3) and carries the measuring section (4), and the second part (1b) is at least partially covered by a hardened sealing material (11), wherein the housing (3) and the semiconductor chip (1) are arranged on a substrate (2) and the semiconductor chip (1) is connected to the substrate (2) by means of the bond wires, the housing (3) comprises a top wall (7), side walls (3a - 3d) extending transversely to the top wall and a barrier section (12) extending as a partition wall between opposite side walls (3c, 3d), wherein the barrier section (12) extends towards the semiconductor chip (1), divides the semiconductor chip (1) into the at least two parts (1a, 1b) forms a barrier for the sealing material, and wherein the measuring section (4) is arranged between the substrate (2) and the top wall (7) and faces the top wall (7).

2. Sensor of claim 1 **characterized in that** the barrier section (12) approaches the semiconductor chip (1) up to a gap (14) and that the gap (14) is filled by the sealing material (11).

3. Sensor of any of the preceding claims **characterized in that** the sealing material (11) covers at least a part of the substrate (2).

4. Sensor of claim 3 **characterized in that** the sealing material (11) connects the substrate (2) to the housing (3).

5. Sensor of any of the claims 3 or 4 **characterized in that** the sealing material (11) covers the bond wires (6) between the semiconductor chip (1) and the substrate (2), and preferably also an integrated circuit (5) arranged on the semiconductor chip (1).

6. Sensor of any of the preceding claims **characterized in that** the semiconductor chip (1) is arranged on a substrate (2) and the side walls (3a- 3d) are resting on a margin section of the substrate (2) and at least a part of the barrier section (12) is set back compared to the side walls for providing space for the semiconductor chip (1).

7. Sensor of any of the preceding claims **characterized in that** the housing (3) is formed by a single part, and in particular that it is of conducting plastics.

8. Sensor of any of the preceding claims **characterized in that** the housing (3) is of plastics and is coated by a conductive layer.

9. Sensor of any of the preceding claims **characterized in that** the housing (3) comprises a filling opening (10) for the sealing material (11).

10. Sensor of any of the preceding claims **characterized in that** the barrier section (12) is designed as partitioning wall in the housing.

11. Sensor of any of the preceding claims **characterized in that** the housing extends around the outer edges of the semiconductor chip.

12. Method for producing a sensor, in particular of any of the preceding claims, wherein the sensor comprises a measuring section (4) on a semiconductor chip (1), is connected to bond wires (6), and is protected by a housing (3), wherein the semiconductor chip (1) is divided into at least two parts (1a, 1b), wherein the first part (1a) is connected through an opening (8) in the housing (3) to the outside and carries the measuring section (4), and the second part (1b) is covered at least partially by a hardening sealing material (11) wherein the semiconductor chip is connected with the bond wires to a substrate (2), the housing (3) comprises a top wall (7), side walls (3a-3d) extending transversely to the top wall and a barrier section (12) formed by a partitioning wall between opposite side walls (3c, 3d), wherein the barrier section (12) extends towards the semiconductor chip (1) and is used for preventing the sealing material (11) from flowing into the first part (1a), and wherein the measuring section (4) is arranged between the substrate (2) and the top wall (7) and faces the top wall (7).

13. Method of claim 12 **characterized in that** the hardening sealing material (11) is brought into a gap (14) between the barrier section (12) and the semiconductor chip (1) by means of capillary forces.

14. Method of any of the claims 12 or 13 **characterized in that** several semiconductor chips (1) are arranged on a substrate matrix (22), that a housing matrix (23) of several, interconnected housings is brought onto the semiconductor chips, that in the area of each sensor sealing material (11) is introduced, and that the substrate matrix (22) is cut up together with the housing matrix (23) for forming the individual sensors.

15. Method of claim 14 **characterized in that** the sensors are brought into a reference environment and calibrated before being cut up.

## Revendications

1. Capteur, en particulier pour la détermination de substances dans un gaz, avec un dispositif de mesure (4) sur une puce de semi-conducteur (1), dans lequel la puce de semi-conducteur (1) est reliée avec des fils de liaison (6) et protégée par un boîtier (3), dans lequel la puce de semi-conducteur (1) est divisée en au moins deux parties (1a, 1b), dans lequel la première partie (1a) est reliée à l'extérieur par au moins une ouverture (8) dans le boîtier (3) et porte le dispositif de mesure (4) et la seconde partie (1b) est recouverte au moins partiellement par une masse d'étanchéité durcie (11), dans lequel le boîtier (3) et la puce de semi-conducteur (1) sont placés sur un substrat (2) et la puce de semi-conducteur (1) est reliée au substrat (2) au moyen des fils de liaison, le boîtier (3) comporte une paroi de recouvrement (7), des parois latérales (3a à 3d) perpendiculaires à la paroi de recouvrement et une partie de barrière (12) sous forme d'une nervure entre des parois latérales opposées (3c, 3d), la partie de barrière (12) s'étend contre la puce de semi-conducteur (1), divise la puce de semi-conducteur (1) en les au moins deux parties (1a, 1b) et forme une barrière pour la masse d'étanchéité, et dans lequel le dispositif de mesure (4) est disposé entre le substrat (2) et la paroi de recouvrement (7) et est orienté vers la paroi de recouvrement (7).

2. Capteur selon la revendication 1, **caractérisé en ce que** la partie de barrière (12) s'approche de la puce de semi-conducteur (1) jusqu'à une fente (14) et **en ce que** la fente (14) est remplie par la masse d'étanchéité (11).

3. Capteur selon l'une quelconque des revendications précédentes, **caractérisé en ce que** la masse d'étanchéité (11) recouvre au moins une partie du substrat (2).

4. Capteur selon la revendication 3, **caractérisé en ce que** la masse d'étanchéité (11) relie le substrat (2) au boîtier (3).

5. Capteur selon l'une quelconque des revendications 3 ou 4, **caractérisé en ce que** la masse d'étanchéité (11) recouvre les fils de liaison (6) entre la puce de semi-conducteur (1) et le substrat (2) et, de préférence, également un circuit intégré (5) placé sur la puce de semi-conducteur (1).

6. Capteur selon l'une quelconque des revendications précédentes, **caractérisé en ce que** la puce de semi-conducteur (1) est placée sur un substrat (2) et les parois latérales (3a à 3d) sont posées sur une zone de bord du substrat (2) et au moins une partie de la partie de barrière (12) est en retrait par rapport aux parois latérales pour faire de la place pour la puce de semi-conducteur (1)

7. Capteur selon l'une quelconque des revendications précédentes, **caractérisé en ce que** le boîtier (3) est fait en une seule partie et, en particulier, **en ce qu'**il est fait en une matière plastique conductrice.

8. Capteur selon l'une quelconque des revendications précédentes, **caractérisé en ce que** le boîtier (3) est fait en matière plastique et est revêtu d'une couche conductrice.

9. Capteur selon l'une quelconque des revendications précédentes, **caractérisé en ce que** le boîtier (3) comporte une ouverture de remplissage (10) pour la masse d'étanchéité (11).

10. Capteur selon l'une quelconque des revendications précédentes, **caractérisé en ce que** la partie de barrière (12) est conçue comme une nervure dans le boîtier.

11. Capteur selon l'une quelconque des revendications précédentes, **caractérisé en ce que** le boîtier (3) s'étend autour des arêtes extérieures de la puce de semi-conducteur (1).

12. Procédé pour la fabrication d'un capteur selon l'une quelconque des revendications précédentes, dans lequel le capteur comporte un dispositif de mesure (4) sur une puce de semi-conducteur (1), est relié avec des fils de liaison (6) et est protégé par un boîtier (3), dans lequel la puce de semi-conducteur (1) est divisée en au moins deux parties (1a, 1b), dans lequel la première partie (1a) est reliée à l'extérieur par au moins une ouverture (8) dans le boîtier (3) et porte le dispositif de mesure (4) et la seconde partie (1b) est recouverte au moins partiellement par une masse d'étanchéité durcie (11), dans lequel la puce de semi-conducteur (1) est reliée au substrat (2) au moyen des fils de liaison (6), le boîtier (3) comporte une paroi de recouvrement (7), des parois latérales (3a à 3d) perpendiculaires à la paroi de recouvrement et une partie de barrière (12) sous forme d'une nervure entre des parois latérales opposées (3c, 3d), la partie de barrière (12) s'étend contre la puce de semi-conducteur (1) et est utilisée pour empêcher un étalement de la masse d'étanchéité (11) dans la première partie (1a), et dans lequel le dispositif de mesure (4) est disposé entre le substrat (2) et la paroi de recouvrement (7) et est orienté vers la paroi de recouvrement (7).

13. Procédé selon la revendication 12, **caractérisé en ce que** la masse d'étanchéité (11) à durcir est insérée par la force capillaire dans une fente (14) entre la partie de barrière (12) et la puce de semi-conducteur (1).

14. Procédé selon l'une quelconque des revendications 12 ou 13, **caractérisé en ce que** plusieurs puces de semi-conducteur (1) sont placées sur une matrice de substrat (22), **en ce qu'**une matrice de boîtier (23) constituée de plusieurs boîtiers reliés les uns aux autres est posée sur les puces de semi-conducteur, **en ce qu'**une masse d'étanchéité (11) est placée dans la zone de chaque capteur et **en ce que** la matrice de substrat (22) est divisée conjointement avec la matrice de boîtier (23) pour former les différents capteurs.

15. Procédé selon la revendication 14, **caractérisé en ce que** les capteurs sont placés et étalonnés dans un milieu de référence avant d'être divisés.
